(19) Europäisches Patentamt
European Patent Office
Office européen des brevets

(11) EP 2 739 150 B1

(12) EUROPÄISCHE PATENTSCHRIFT

(45) Veröffentlichungstag und Bekanntmachung des Hinweises auf die Patenterteilung:
**19.10.2016 Patentblatt 2016/42**

(21) Anmeldenummer: **12743891.9**

(22) Anmeldetag: **31.07.2012**

(51) Int Cl.:
***A01N 25/04*** *(2006.01)* ***A01N 43/90*** *(2006.01)*
***A01P 1/00*** *(2006.01)* ***A61K 8/49*** *(2006.01)*
***A61Q 19/00*** *(2006.01)* ***C11D 1/66*** *(2006.01)*
***C11D 3/20*** *(2006.01)* ***C11D 3/22*** *(2006.01)*

(86) Internationale Anmeldenummer:
**PCT/EP2012/003250**

(87) Internationale Veröffentlichungsnummer:
**WO 2013/017261 (07.02.2013 Gazette 2013/06)**

(54) **ZUSAMMENSETZUNG ENTHALTEND ISOSORBIDMONOESTER UND ISOSORBIDDIESTER**

COMPOSITION COMPRISING ISOSORBIDE MONOESTER AND ISOSORBIDE DIESTER

COMPOSITION COMPRENANT DU MONOESTER D'ISOSORBIDE ET DU DIESTER DE ISOSORBIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.08.2011 DE 102011109498**

(43) Veröffentlichungstag der Anmeldung:
**11.06.2014 Patentblatt 2014/24**

(73) Patentinhaber: **Clariant International Ltd**
**4132 Muttenz (CH)**

(72) Erfinder:
- **PILZ, Maurice Frederic**
  **60329 Frankfurt am Main (DE)**
- **KLUG, Peter**
  **63762 Großostheim (DE)**
- **SCHERL, Franz-Xaver**
  **84508 Burgkirchen (DE)**
- **GROHMANN, Joerg**
  **65527 Niedernhausen (DE)**

(74) Vertreter: **Holmes, Rosalind et al**
**Clariant Produkte (Deutschland) GmbH**
**Patent & License Management Chemicals**
**Industriepark Höchst, G 860**
**65926 Frankfurt am Main (DE)**

(56) Entgegenhaltungen:
**WO-A2-2010/108738** **WO-A2-2010/136121**
**US-A1- 2011 117 036**

- **PETER STOSS ET AL: "Regioselektive Acylierung von 1,4:3,6-Dianhydro-D-glucit", SYNTHESIS, Bd. 1987, Nr. 02, 1. Januar 1987 (1987-01-01), Seiten 174-176, XP55039551, ISSN: 0039-7881, DOI: 10.1055/s-1987-27878**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 739 150 B1

## Beschreibung

Die vorliegende Erfindung betrifft Zusammensetzungen enthaltend Isosorbidmonoester und Isosorbiddiester sowie deren Verwendung zur Herstellung von kosmetischen, dermatologischen oder pharmazeutischen Zusammensetzungen, Pflanzenschutzformulierungen, Wasch- oder Reinigungsmitteln oder Farb- oder Anstrichmitteln.

In der Industrie existiert eine große Auswahl an Verdickern, die verwendet werden können, um die Viskosität von Produkten wie beispielsweise von kosmetischen, dermatologischen oder pharmazeutischen Zusammensetzungen, Pflanzenschutzformulierungen, Wasch- oder Reinigungsmitteln oder Farb- oder Anstrichmitteln auf ein gewünschtes Maß einzustellen.

Entsprechend sind auch zahlreiche Konservierungsmittel oder Biozide bekannt, die verwendet werden können, um derartige Produkte gegen mikrobiellen Befall zu schützen. Beispielsweise können Konservierungsmittel aus Annex V der EU-Kosmetik-Richtlinie oder Biozide der EU-Biozid-Richtlinie hierfür verwendet werden.

Nachteilig an der Verwendung vieler Verdicker und Konservierungsmittel ist jedoch, dass ihre Herstellung oftmals aufwändig ist und auf synthetischen Rohstoffen basiert. Zudem ist ihre verdickende oder konservierende Wirkung oftmals verbesserungsbedürftig, so dass hohe Einsatzkonzentrationen für eine ausreichende Verdickung und Konservierung notwendig sind.

In der Industrie besteht zudem ein gesteigertes Interesse an Substanzen bzw. Zusammensetzungen, die bereits die genannten Eigenschaften in sich vereinigen, d. h. die sowohl eine vorteilhafte Verdickungsleistung als auch eine vorteilhafte Konservierungsmittelleistung aufweisen.

Es bestand die Aufgabe, Substanzen bzw. Zusammensetzungen zur Verfügung zu stellen, die eine vorteilhafte Verdickungsleistung aufweisen und sich zudem durch den Vorteil auszeichnen, dass sie auf nachwachsenden Rohstoffen basieren. Vorzugsweise sollten diese Substanzen bzw. Zusammensetzungen darüber hinaus auch eine vorteilhafte Konservierungsmittelleistung aufweisen.

Überraschend wurde nun gefunden, dass diese Aufgabe gelöst wird durch Zusammensetzungen enthaltend eine oder mehrere Verbindungen der Formel (I) und eine oder mehrere Verbindungen der Formel (II)

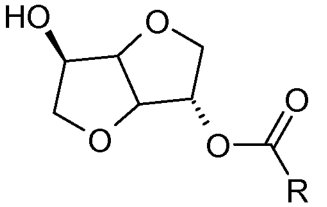

(I)

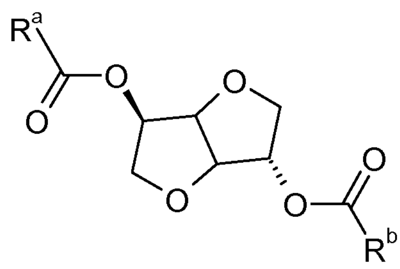

(II)

worin

R, $R^a$ und $R^b$ jeweils unabhängig voneinander lineare oder verzweigte, gesättigte Alkylgruppen mit 5 bis 11, vorzugsweise 7 bis 9 und besonders bevorzugt 7 Kohlenstoffatomen oder lineare oder verzweigte, ein- oder mehrfach ungesättigte Alkenylgruppen mit 5 bis 11, vorzugsweise 7 bis 9 und besonders bevorzugt 7 Kohlenstoffatomen sind, und wobei die Gesamtmenge der Verbindungen der Formeln (I) und (II), bezogen auf das Gesamtgewicht der Zusammensetzung, mindestens 60 Gew.-% beträgt,

wobei die Zusammensetzung zusätzlich zu den Verbindungen der Formeln (I) und (II) eine oder mehrere andere Substanzen ausgewählt aus der Gruppe bestehend aus Sorbitol, Sorbitolestern, Sorbitan, Sorbitanestern, Isosorbid und Carbonsäuren enthält

und die OH-Zahl der Mischung aus den Verbindungen der Formeln (I) und (II) und zusätzlich der einen oder den mehreren Verbindungen ausgewählt aus der Gruppe bestehend aus Sorbitol, Sorbitolestern, Sorbitan, Sorbitanestern, Isosorbid und Carbonsäuren kleiner oder gleich 250 ist,

wobei die Zusammensetzung 0,4 bis 0,8 Gewichtsteile an der einen oder den mehreren Verbindungen der Formel (II), bezogen auf 1,0 Gewichtsteile an der einen oder den mehreren Verbindungen der Formel (I), enthält.

Gegenstand der Erfindung sind somit Zusammensetzungen wie vorstehend beschrieben.

Die erfindungsgemäßen Zusammensetzungen weisen sowohl eine vorteilhafte Verdickungsleistung als auch eine vorteilhafte Konservierungsmittelleistung, vorzugsweise gegenüber Hefen und Pilzen und besonders bevorzugt gegenüber Pilzen, auf und zeichnen sich zudem durch den Vorteil aus, dass sie auf nachwachsenden Rohstoffen basieren.

Die Verbindungen der Formeln (I) und (II) weisen eine sehr gute Verdickungsleistung auf und basieren auf nachwachsenden Rohstoffen. Darüber hinaus weisen insbesondere die Verbindungen der Formel (I) auch eine sehr gute Konservierungsmittelleistung auf und wirken vorzugsweise als Fungizide. Dies bedeutet im Rahmen der vorliegenden Erfindung, dass die Verbindungen der Formel (I) vorzugsweise als antimikrobielle Wirkstoffe gegen Hefen und Pilze wirken. Die Verbindungen der Formel (I) wirken in einer besonders bevorzugten Ausführungsform der Erfindung als antimikrobielle Wirkstoffe gegen Pilze.

Im Vergleich zur Verwendung von organischen Säuren als Konservierungsmittel besitzen die Verbindungen der Formel (I) zudem den Vorteil einer Wirksamkeit über einen breiteren pH-Wert-Bereich. Während organische Säuren oftmals eine gute Wirkung nur im pH-Wert-Bereich von 3,5 bis 6 zeigen, können die Verbindungen der Formel (I) auch bei höheren pH-Werten vorteilhaft eingesetzt werden.

Zusammensetzungen, die zumindest zum Teil auf nachwachsenden Rohstoffen basieren und z. B. als Konservierungsmittel oder Verdicker verwendet werden können, sind bereits bekannt.

WO 2010/108738 A2 (Evonik) beschreibt Formulierungen zur Reinigung und Pflege von menschlichen oder tierischen Körperteilen enthaltend Sorbitancarbonsäureester, wobei sich der Carbonsäureanteil des Sorbitancarbonsäureesters ableitet von einer Carbonsäure enthaltend 6 bis 10 Kohlenstoffatome und die Sorbitancarbonsäureester eine Hydroxylzahl (OH-Zahl) von größer 350 aufweisen sowie die Verwendung der genannten Sorbitancarbonsäureester als Viskositätsregler, Pflegewirkstoff, Schaumbooster oder Solubilisator in reinigenden oder pflegenden Formulierungen.

DE 10 2009 022 444 (Clariant) beschreibt flüssige Zusammensetzungen enthaltend Sorbitanmonocaprylat und antimikrobielle Wirkstoffe wie z. B. spezielle organische Säuren und ihre Salze, spezielle Formaldehyd-Donoren, spezielle Isothiazolinone, spezielle Parabenester und ihre Salze und spezielle Pyridone und ihre Salze sowie ihre Verwendung zum Konservieren von kosmetischen, dermatologischen oder pharmazeutischen Produkten.

In der DE 10 2009 022 445 (Clariant) werden flüssige Zusammensetzungen enthaltend Sorbitanmonocaprylat und Alkohol und ihre Verwendung zum Konservieren von kosmetischen, dermatologischen oder pharmazeutischen Produkten offenbart.

In der JP 8187070 (A) (Lion) wird eine Mischung aus Fettsäuremonoestern von $C_8$-$C_{18}$ Fettsäuren und mindestens einem Polyol ausgewählt aus Sorbitol, 1,5-Sorbitan, 1,4-Sorbitan und Isosorbid und Fettsäurediestern dieser Fettsäuren und Polyole in einem Gewichtsverhältnis von Monoester: Diester von 33 : 7 bis 9 : 1 als antimikrobieller Wirkstoff gegen Bakterien für Nahrungsmittel oder Getränke offenbart.

JP 8173787 (A) (Lion) beschreibt eine Zusammensetzung enthaltend einen oberflächenaktiven Stoff enthaltend einen Fettsäureester von dehydratisiertem Sorbitol Die Zusammensetzungen können Mono- oder Diester von Capryl- und/oder Caprinsäure mit einem Polyol ausgewählt aus der Gruppe bestehend aus 1,5-Sorbitan, 1,4-Sorbitan und Isosorbid enthalten. Es wird insbesondere die Verwendung der Zusammensetzungen als Öl-in-Wasser-Emulgator und als Reinigungsbasis beschrieben.

Verbindungen der Formeln (I) und (II) können z. B. nach dem Fachmann geläufigen Methoden hergestellt werden. Beispielsweise können die Verbindungen der Formeln (I) und (II) durch Veresterung von Isosorbid nach üblichen und dem Fachmann bekannten Methoden hergestellt werden, wobei sowohl Isosorbid selbst als auch die zur Veresterung verwendeten Säurekomponenten wiederum käuflich erwerbbar sind.

Vorzugsweise sind die Reste R, $R^a$ und $R^b$ jeweils unabhängig voneinander in den Verbindungen der Formeln (I) und (II) lineare gesättigte Alkylreste mit 7 bis 9 Kohlenstoffatomen.

Besonders bevorzugt sind die Reste R, $R^a$ und $R^b$ in den Verbindungen der Formeln (I) und (II) lineare gesättigte Alkylreste mit 7 Kohlenstoffatomen.

Die erfindungsgemäßen Zusammensetzungen enthalten 0,4 bis 0,8 Gewichtsteile an der einen oder den mehreren Verbindungen der Formel (II), wobei es sich dabei vorzugsweise um Isosorbiddicaprylat handelt, bezogen auf 1,0 Gewichtsteile an der einen oder den mehreren Verbindungen der Formel (I) und vorzugsweise bezogen auf 1,0 Gewichtsteile an Isosorbidmonocaprylat.

Unter den soeben genannten Zusammensetzungen sind diejenigen bevorzugt, die 0,05 bis 0,7, vorzugsweise 0,1 bis 0,6 und besonders bevorzugt 0,2 bis 0,5 Gewichtsteile Isosorbid, bezogen auf 1,0 Gewichtsteile an der einen oder den mehreren Verbindungen der Formel (I) und vorzugsweise bezogen auf 1,0 Gewichtsteile an Isosorbidmonocaprylat, enthalten.

In einer weiteren bevorzugten Ausführungsform der Erfindung enthalten die erfindungsgemäßen Zusammensetzungen 0,001 bis 0,2, vorzugsweise 0,01 bis 0,15 und besonders bevorzugt 0,05 bis 0,13 Gewichtsteile an der einen oder den mehreren Verbindungen der Formel (I), wobei es sich dabei vorzugsweise um Isosorbidmonocaprylat handelt, bezogen auf 1,0 Gewichtsteile an der einen oder den mehreren Verbindungen der Formel (II) und vorzugsweise bezogen

auf 1,0 Gewichtsteile an Isosorbiddicaprylat.

Die erfindungsgemäßen Zusammensetzungen enthalten zusätzlich zu der einen oder den mehreren Verbindungen der Formel (I) und der einen oder den mehreren Verbindungen der Formel (II) eine oder mehrere andere Substanzen ausgewählt aus der Gruppe bestehend aus Sorbitol, Sorbitolestern (bei Sorbitolestern kann es sich um Mono-, Di-, Tri-, Tetra-, Penta- und/oder Hexaester handeln), Sorbitan, Sorbitanestern (bei Sorbitanestern kann es sich um Mono-, Di-, Tri- und/oder Tetraester handeln), Isosorbid und Carbonsäuren. Bei "Sorbitan" kann es sich beispielsweise um 1,4- oder 1,5-Sorbitan handeln. Sowohl die Carbonsäuren selbst als auch die den Säurekomponenten der genannten Ester zu Grunde liegenden Carbonsäuren entsprechen der Formel $R^cCOOH$, worin $R^c$ die bei den Formeln (I) und (II) für R, $R^a$ und $R^b$ angegebenen Bedeutungen besitzt und vorzugsweise ein linearer gesättigter Alkylrest mit 7 Kohlenstoffatomen ist, d.h. die Carbonsäure $R^cCOOH$ vorzugsweise Caprylsäure ist.

Die OH-Zahl der Mischung aus den Verbindungen der Formeln (I) und (II) und zusätzlich der einen oder den mehreren Verbindungen ausgewählt aus der Gruppe bestehend aus Sorbitol, Sorbitolestern, Sorbitan, Sorbitanestern, Isosorbid und Carbonsäuren ist in der Zusammensetzung kleiner oder gleich 250, vorzugsweise kleiner oder gleich 230, besonders bevorzugt kleiner oder gleich 210 und insbesondere bevorzugt kleiner oder gleich 100.

In einer weiteren bevorzugten Ausführungsform der Erfindung enthalten die erfindungsgemäßen Zusammensetzungen entweder keine Carbonsäure $R^cCOOH$ oder bis zu 0,1, bevorzugt 0,0001 bis 0,05 und besonders bevorzugt 0,001 bis 0,01 Gewichtsteile Carbonsäure $R^cCOOH$, wobei $R^c$ die oben bei den Formeln (I) und (II) für R, $R^a$ und $R^b$ angegebene Bedeutung besitzt, und wobei die Carbonsäure vorzugsweise Caprylsäure ist, bezogen auf 1,0 Gewichtsteile der Gesamtmenge an der einen oder den mehreren Verbindungen der Formel (I) und der einen oder den mehreren Verbindungen der Formel (II) und vorzugsweise bezogen auf 1,0 Gewichtsteile der Gesamtmenge an Isosorbidmonocaprylat und Isosorbidd icaprylat.

In einer weiteren besonders bevorzugten Ausführungsform der Erfindung enthalten die erfindungsgemäßen Zusammensetzungen keine Verbindungen ausgewählt aus Sorbitol und Sorbitolestern. Sofern die erfindungsgemäßen Zusammensetzungen jedoch eine oder mehrere Verbindungen ausgewählt aus Sorbitol und Sorbitolestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 5,0 Gew.-%, besonders bevorzugt in einer Menge kleiner oder gleich 3,0 Gew.-%, insbesondere bevorzugt in einer Menge kleiner oder gleich 1,0 Gew.-% und außerordentlich bevorzugt in einer Menge kleiner oder gleich 0,5 Gew.-% in den erfindungsgemäßen Zusammensetzungen enthalten, wobei die Angaben in Gew.-% jeweils auf das Gesamtgewicht der fertigen erfindungsgemäßen Zusammensetzungen bezogen sind.

In einer weiteren besonders bevorzugten Ausführungsform der Erfindung enthalten die erfindungsgemäßen Zusammensetzungen keine Verbindungen ausgewählt aus Sorbitan und Sorbitanestern. Sofern die erfindungsgemäßen Zusammensetzungen jedoch eine oder mehrere Verbindungen ausgewählt aus Sorbitan und Sorbitanestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 20,0 Gew.-%, besonders bevorzugt in einer Menge kleiner oder gleich 10,0 Gew.-%, insbesondere bevorzugt in einer Menge kleiner oder gleich 5,0 Gew.-% und außerordentlich bevorzugt in einer Menge kleiner oder gleich 1,0 Gew.-% in den erfindungsgemäßen Zusammensetzungen enthalten, wobei die Angaben in Gew.-% jeweils auf das Gesamtgewicht der fertigen erfindungsgemäßen Zusammensetzungen bezogen sind.

In einer weiteren besonders bevorzugten Ausführungsform der Erfindung enthalten die erfindungsgemäßen Zusammensetzungen jeweils eine oder mehrere Verbindungen der Formeln (I) und (II) und zusätzlich einen oder mehrere Sorbitanester aus Sorbitan und Carbonsäuren $R^cCOOH$, vorzugsweise ausgewählt aus Sorbitanestern aus 1,4- und/oder 1,5-Sorbitan und Carbonsäuren $R^cCOOH$, wobei $R^c$ eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, und wobei das Gewichtsverhältnis der Verbindungen der Formeln (I) und (II) gemeinsam zu dem einen oder den mehreren soeben genannten Sorbitanestern von 70 : 30 bis 100 : 0, vorzugsweise von 80 : 20 bis 100 : 0, besonders bevorzugt von 90 : 10 bis 100 : 0 und insbesondere bevorzugt von 95 : 5 bis 100 : 0 ist. Das angegebene Gewichtsverhältnis von "100 : 0" bedeutet, dass die soeben genannten erfindungsgemäßen Zusammensetzungen in dieser besonders bevorzugten Ausführungsform der Erfindung keinen Sorbitanester enthalten müssen.

Unter den soeben genannten erfindungsgemäßen Zusammensetzungen sind solche bevorzugt, worin der eine oder die mehreren Sorbitanester aus Sorbitan und Carbonsäuren $R^cCOOH$ ausgewählt sind aus Sorbitanestern aus Sorbitan und Caprylsäure, vorzugsweise ausgewählt sind aus Sorbitanestern aus 1,4- und/oder 1,5-Sorbitan und Caprylsäure und besonders bevorzugt ausgewählt sind aus der Gruppe bestehend aus Sorbitanmonocaprylat und Sorbitandicaprylat.

Unter der Hydroxyl- oder OH-Zahl einer Substanz wird diejenige Menge KOH in mg verstanden, die der bei der Acetylierung von 1 g Substanz gebundenen Menge an Essigsäure äquivalent ist.

Geeignete Bestimmungsmethoden zur Ermittlung der OH-Zahl sind z. B. DGF C-V 17 a (53), Ph. Eur. 2.5.3

Method A und DIN 53240.

Im Rahmen der vorliegenden Erfindung werden die OH-Zahlen in Anlehnung an DIN 53240-2 bestimmt. Hierbei wird wie folgt vorgegangen: Es wird 1 g auf 0,1 mg genau von der homogenisierten zu messenden Probe eingewogen. 20,00 ml Acetylierungsgemisch (Acetylierungsgemisch: in 1 Liter Pyridin werden 50 ml Essigsäureanhydrid eingerührt) werden zugeben. Die Probe wird vollständig im Acetylierungsgemisch gelöst, gegebenenfalls unter Rühren und Erwärmen. 5 ml Katalysatorlösung (Katalysatorlösung: 2 g 4-Dimethylaminopyridin werden in 100 ml Pyridin gelöst) werden zugeben. Das Reaktionsgefäß wird verschlossen und 10 Minuten in das auf 55 °C vorgeheizte Wasserbad gestellt und dabei durchmischt. Die Reaktionslösung wird danach mit 10 ml vollentsalztem Wasser versetzt, das Reaktionsgefäß erneut verschlossen und nochmals im Schüttelwasserbad 10 Minuten abreagieren gelassen. Die Probe wird auf Raumtemperatur (25 °C) abgekühlt. Anschließend werden 50 ml 2-Propanol und 2 Tropfen Phenolphthalein zugeben. Diese Lösung wird mit Natronlauge (Natronlauge c = 0,5 mol/l) titriert (Va). Unter den gleichen Bedingungen, jedoch ohne Probeneinwaage, wird der Wirkwert des Acetylierungsgemisches bestimmt (Vb).

Aus dem Verbrauch der Wirkwertbestimmung und der Titration der Probe wird die OH-Zahl (OHZ) nach folgender Formel berechnet:

$$OHZ = \frac{(Vb - Va) \cdot c \cdot t \cdot M}{E}$$

OHZ = Hydroxylzahl in mg KOH/g Substanz
Va = Verbrauch an Natronlauge in ml bei der Titration der Probe
Vb = Verbrauch an Natronlauge in ml bei der Titration des Wirkwertes
c = Stoffmengenkonzentration der Natronlauge in mol/l
t = Titer der Natronlauge
M = Molare Masse von KOH = 56,11 g/mol
E = Probeneinwaage in g

(Vb - Va) ist diejenige Menge der verwendeten Natronlauge in ml, die der bei der oben beschriebenen Acetylierung der zu messenden Probe gebundenen Menge an Essigsäure äquivalent ist.

Die soeben beschriebene Methode zur Bestimmung der OH-Zahl wird im Folgenden als "Methode OHZ-A" bezeichnet.

Die erfindungsgemäßen Zusammensetzungen sind in vorteilhafter Weise zur Herstellung von kosmetischen, dermatologischen oder pharmazeutischen Zusammensetzungen, Pflanzenschutzformulierungen, Wasch- oder Reinigungsmitteln oder Farb- oder Anstrichmitteln geeignet.

Weiterer Gegenstand der Erfindung ist daher die Verwendung einer erfindungsgemäßen Zusammensetzung zur Herstellung von kosmetischen, dermatologischen oder pharmazeutischen Zusammensetzungen, Pflanzenschutzformulierungen, Wasch- oder Reinigungsmitteln oder Farb- oder Anstrichmitteln.

Die mit Hilfe der erfindungsgemäßen Zusammensetzungen hergestellten kosmetischen, dermatologischen oder pharmazeutischen Zusammensetzungen, Pflanzenschutzformulierungen, Wasch- oder Reinigungsmittel und Farb- oder Anstrichmittel enthalten die eine oder die mehreren Verbindungen der Formel (I) und die eine oder die mehreren Verbindungen der Formel (II) gemeinsam vorzugsweise in Mengen von 0,01 bis 10,0 Gew.-%, besonders bevorzugt in Mengen von 0,1 bis 5,0 Gew.-% und insbesondere bevorzugt in Mengen von 0,2 bis 3,0 Gew.-%, jeweils bezogen auf das Gesamtgewicht der fertigen kosmetischen, dermatologischen oder pharmazeutischen Zusammensetzungen, Pflanzenschutzformulierungen, Wasch- oder Reinigungsmittel oder Farb- oder Anstrichmittel.

Die kosmetischen, dermatologischen oder pharmazeutischen Zusammensetzungen, die Pflanzenschutzformulierungen, die Wasch- oder Reinigungsmittel oder die Farb- oder Anstrichmittel haben Viskositäten vorzugsweise im Bereich von 50 bis 200 000 mPa · s, besonders bevorzugt im Bereich von 500 bis 100 000 mPa · s, insbesondere bevorzugt im Bereich von 2 000 bis 50 000 mPa · s und außerordentlich bevorzugt im Bereich von 5 000 bis 30 000 mPa · s (20 °C, Brookfield RVT, RV-Spindel-Satz bei 20 Umdrehungen pro Minute).

Die kosmetischen, dermatologischen oder pharmazeutischen Zusammensetzungen liegen vorzugsweise in Form von Fluids, Gelen, Schäumen, Sprays, Lotions oder Cremes vor.

Die kosmetischen, dermatologischen oder pharmazeutischen Zusammensetzungen, die Pflanzenschutzformulierungen, die Wasch- oder Reinigungsmittel oder die Farb- oder Anstrichmittel sind vorzugsweise auf wässriger oder wässrig-alkoholischer Basis aufgebaut oder liegen als Emulsionen oder Dispersionen vor. Besonders bevorzugt liegen sie als Emulsionen vor und insbesondere bevorzugt liegen sie als Öl-in-Wasser Emulsionen vor.

Die kosmetischen, dermatologischen oder pharmazeutischen Zusammensetzungen, die Pflanzenschutzformulierungen, die Wasch- oder Reinigungsmittel oder die Farb- oder Anstrichmittel können als weitere Hilfs- und Zusatzstoffe alle üblicherweise für die jeweilige Anwendung verwendeten Substanzen enthalten, beispielsweise Öle, Wachse, Emulgatoren, Co-Emulgatoren, Dispergatoren, Tenside, Entschäumer, Solubilisatoren, Elektrolyte, Hydroxysäuren, Stabilisatoren, Polymere, Filmbildner, weitere Verdicker, Gelierungsmittel, Überfettungsmittel, Rückfetter, weitere antimikrobielle Wirkstoffe, biogene Wirkstoffe, Adstringentien, Aktivstoffe, deodorierende Stoffe,

Sonnenschutzfilter, Antioxidantien, Oxidantien, Feuchthaltemittel, Lösungsmittel, Farbmittel, Pigmente, Perlglanzmittel, Duftstoffe, Trübungsmittel und/oder Silikone.

Die kosmetischen, dermatologischen oder pharmazeutischen Zusammensetzungen, die Pflanzenschutzformulierungen, die Wasch- oder Reinigungsmittel oder die Farb- oder Anstrichmittel besitzen pH-Werte von vorzugsweise 2 bis 11, besonders bevorzugt von 4,5 bis 8,5 und insbesondere bevorzugt von 5,5 bis 6,5.

Die nachfolgenden Beispiele und Anwendungen sollen die Erfindung näher erläutern. Bei allen Prozentangaben handelt es sich um Gewichts-% (Gew.-%), sofern nicht explizit anders angegeben. Versuchsbeispiele:

A) Herstellung von Isosorbidcaprylat 1

In einer Rührapparatur mit Destillationsaufsatz werden 190,0 g (1,3 mol) Isosorbid ("Sorbon" von Ecogreen Oleochemicals) und 187,5 g (1,3 mol) Octansäure (Caprylsäure) bei 80 °C zusammen mit 0,38 g Natronlauge (18 Gew.-%ig, wässrig) als Katalysator vorgelegt. Unter Rühren und Stickstoffüberleitung (10 - 12 Liter pro Stunde) wird das Reaktionsgemisch zunächst auf 180 °C aufgeheizt, wobei das Reaktionswasser abzudestillieren beginnt. Der Ansatz wird dann in 1 Stunde auf 190 °C und in weiteren 2 Stunden auf 210 °C aufgeheizt. Nach Erreichen von 210 °C wird solange verestert bis eine Säurezahl von < 1 mg KOH/g erreicht ist. Man erhält 345,7 g bernsteinfarbenes Isosorbidcaprylat (97 % der Theorie). Der pH-Wert (5 Gew.-% in Ethanol/Wasser 1:1) beträgt 5,9. Der pH-Wert wurde gemessen gemäß DIN EN 1262.

Weitere analytische Kenndaten des Isosorbidcaprylats 1:

| | |
|---|---|
| Säurezahl: | 0,9 mg KOH/g, gemessen gemäß DIN EN ISO 2114 |
| Hydroxyl-Zahl: | 206 mg KOH/g, gemessen in Anlehnung an DIN 53240-2 nach Methode OHZ-A |
| Verseifungszahl: | 204 mg KOH/g, gemessen gemäß DIN EN ISO 3681 |

Das Isosorbidcaprylat 1 besitzt folgende Zusammensetzung:

| Substanz | Gew.-% |
|---|---|
| Caprylsäure | 0,4 |
| Isosorbid | 18,1 |
| Isosorbidmonocaprylat | 50,9 |
| Isosorbidd icaprylat | 30,6 |

B) Herstellung von Isosorbiddicaprylat

In einer 1 Liter-Rührapparatur mit Stickstoffüberleitung werden 219,0 g (1,5 mol) Isosorbid und 461,4 g (3,2 mol) Caprylsäure unter Rühren und Stickstoffüberleitung auf 180 °C erhitzt. Der Reaktionsansatz wird solange auf 180 °C erhitzt, bis kein Reaktionswasser mehr abdestilliert (etwa 28 h). Anschließend wird die Temperatur schrittweise bis auf 210 °C erhöht (insgesamt über etwa 30 h). Die Reaktion ist dann beendet, wenn eine Restsäurezahl von < 2 mg KOH /g erreicht ist. Es wird eine klare, rotbraune Flüssigkeit erhalten.

Weitere analytische Kennzahlen des Reaktionsproduktes:

| | |
|---|---|
| Säurezahl: | 0,8 mg KOH/g gemessen gemäß DIN EN ISO 2114 |
| Hydroxyl-Zahl: | 25,2 mg KOH/g gemessen in Anlehnung an DIN 53240-2 nach Methode OHZ-A |
| Verseifungszahl: | 54,6 mg KOH/g gemessen gemäß DIN EN ISO 3681 |

Zur weiteren Aufreinigung wurde das Produkt bei einem Druck von ≤ 1 mbar und einer Sumpftemperatur von 210 °C bis 240 °C destilliert. Es werden 251,6 g einer gelben klaren Flüssigkeit erhalten.

Das Isosorbiddicaprylat besitzt folgende Zusammensetzung:

| Substanz | Gew.-% |
|---|---|
| Isosorbidmonocaprylat | 9,4 |
| Isosorbidd icaprylat | 89,6 |
| Rest | 1 |

C) Bestimmung der Verdickungsleistung

Unter Verwendung von Genapol® LRO (Sodium Laureth-2 Sulfate, 27 Gew.-% in Wasser) und Genagen® KB (Cocobetain, 30 Gew.-% in Wasser) und zusätzlich Wasser wurde eine Mischung hergestellt, die die beiden Tenside im Gewichtsverhältnis 8 : 2 zu 15 Gew.-% in Wasser enthält (im Folgenden "Mischung A" genannt). Es wurde die Verdickungsleistung von Isosorbidcaprylat 1, Isosorbiddicaprylat und Isosorbid in Mischung A bestimmt. Die Ergebnisse sind in Tabelle 1 dargestellt.

Tabelle 1 gemessene Viskositäten

| Zu Mischung A zugegebene Substanz; Menge [Gew.-%] | Viskosität [mPa · s] |
|---|---|
| Keine | 135 |
| Isosorbidcaprylat 1 [1 Gew.-%] | 2510 |
| Isosorbiddicaprylat [1 Gew.-%] | 2390 |
| Isosorbid [1 Gew.-%] | 160 |

Wie den Ergebnissen der Tabelle 1 zu entnehmen ist, besitzt Isosorbid keine nennenswerte Verdickungsleistung, während Isosorbidcaprylat 1 und Isosorbiddicaprylat eine signifikante Verdickung bewirkt.

D) Bestimmung der antimikrobiellen Wirksamkeit von Isosorbidcaprylat 1

Im Folgenden wird die antimikrobielle Wirksamkeit von Isosorbidcaprylat 1 in Butylpolyglykol gegen Bakterien, Pilze und Hefen untersucht. Für die Ausprüfung mit Bakterien wurde Isosorbidcaprylat 1 mit Butylpolyglykol verdünnt und anschließend zu flüssigem, auf pH 7 (+/- 0,2) gepufferten Caso-Agar (Casein-Pepton-Agar) bei 50 °C in unterschiedlichen Konzentrationen gegeben (im Folgenden Zusammensetzungen B1, B2, etc. genannt). Für die Ausprüfung mit Pilzen und mit Hefen wurde Isosorbidcaprylat 1 mit Butylpolyglykol verdünnt und anschließend zu flüssigem, auf pH 5,6 (+/- 0,2) gepufferten Sabouraud-4 %-Dextrose Agar in unterschiedlichen Konzentrationen gegeben (im Folgenden Zusammensetzungen PH1, PH2, etc. genannt). Jede der Zusammensetzungen B1, B2, etc. bzw. PH1, PH2 etc. wurde in Petrischalen ausgegossen und jeweils mit der gleichen Menge an Bakterien, Pilzen und Hefen beimpft. Die minimale Hemmkonzentration (MHK) ist die Konzentration, bei der eine Hemmung des Wachstums der Bakterien, Pilze und Hefen in den Zusammensetzungen B1, B2, etc. bzw. PH1, PH2, etc. auftritt.

Die ermittelten und im Folgenden in Tabelle 2 angegebenen Werte für die minimalen Hemmkonzentrationen von Isosorbidcaprylat 1 sind bereits um den Verdünnungseffekt des Butylpolyglykols bereinigt.

Tabelle 2 Minimale Hemmkonzentrationen (MHK) von Isosorbidcaprylat 1

| Untersuchte Bakterien (B), Pilze (P) oder Hefen (H) | MHK von Isosorbidcaprylat 1 [ppm] |
|---|---|
| *Staphylococcus aureus (B)* | 2500 |
| *Pseudomonas aeruginosa (B)* | 10000 |
| *Escherichia coli (B)* | 7500 |
| *Enterobacter aerogenes (B)* | 10000 |
| *Klebsiella pneumoniae (B)* | 10000 |
| *Proteus vulgaris (B)* | 5000 |
| *Pseudomonas oleovorans (B)* | 10000 |
| *Citrobacter freundii (B)* | 10000 |

(fortgesetzt)

| Untersuchte Bakterien (B), Pilze (P) oder Hefen (H) | MHK von Isosorbidcaprylat 1 [ppm] |
|---|---|
| *Candida albicans (H)* | 600 |
| *Aspergillus brasiliensis (P)* | 800 |
| *Penicillium minioluteum (P)* | 600 |
| *Aspergillus terreus (P)* | 600 |
| *Fusarium solani (P)* | 600 |
| *Penicillium funicolosium (P)* | 400 |

An den in Tabelle 2 aufgeführten Ergebnissen erkennt man, dass Isosorbidcaprylat 1 eine antimikrobielle Wirksamkeit besitzt, insbesondere gegen die Hefe *Candida albicans* und die getesteten Pilze.

E) Anwendungsbeispiele

Folgenden Formulierungen werden unter Verwendung der erfindungsgemäßen Zusammensetzung Isosorbidcaprylat 1 hergestellt.

Formulierungsbeispiel 1: Revitalisierende Feuchtigkeitscreme

| Phase | Inhaltsstoff | Gew.-% |
|---|---|---|
| A | Hostacerin® SFO<br>Sunflower Seed Oil Sorbitol Esters<br>Velsan® CCT<br>Caprylic/Capric Triglyceride<br>Cetiol® OE<br>Dicaprylyl Ether<br>Lanette® 22<br>Benehyl Alcohol<br>Lanette® 18<br>Stearyl Alcohol<br>Fucogel® 1000<br>Biosacchride Gum-1 | 2,0<br><br>4,5<br><br>4,5<br><br>4,0<br><br>4,0<br><br>1,0 |
| B | Coenzyme® Q 10<br>Ubiquinone | 0,1 |
| C | Wasser<br>Glycerin<br>Hostaphat® CK 100<br>Potassium Cetyl Phosphate | ad 100<br>10,0<br>0,6 |
| D | Phenoxyethanol<br>Isosorbidcaprylat 1 | 1,0<br>1,0 |
| E | NaOH (10 Gew.-%ig in Wasser) | q.s. |

Herstellung:

I Mische die Komponenten von A und erwärme auf 80 °C
II Mische die Komponenten von C und erwärme auf 80 °C
III Gebe B zu I.
IV Gebe II zu III und rühre bis die Mischung auf Raumtemperatur abgekühlt ist

V Gebe D zu IV
VI Stelle den pH-Wert mit E auf pH 5,5 ein

Formulierungsbeispiel 2:

| Phase | Inhaltsstoff | Gew.-% |
|---|---|---|
| A | Hostacerin® EWO<br>Polyglyceryl-2-Sesquiisostearate (and) Cera Alba (and) Carnauba Wax (and) Ethylhexylstearate (and) Magnesium Stearate (and) Aluminum Stearate<br>Isopropyl Palmitate<br>Avocado Oil<br>Velsan® CCT<br>Caprylic/Capric Tryglyceride | 16,0<br><br><br>10,0<br>2,0<br>2,5 |
| B | Octopirox®<br>Piroctone Olamine<br>Propylene Glycol | 0,05<br><br>1,0 |
| C | Wasser<br>Glycerin<br>Magnesium Sulfate * 7 $H_2O$<br>Allantoin | ad 100<br>4,0<br>0,7<br>0,5 |
| D | Tocopheryl Acetate<br>Rosmarinus Officinalis (Rosemary) Leaf Oil<br>Urea<br>Isosorbidcaprylat 1<br>Phenoxyethanol | 0,5<br>0,1<br>10,0<br>1,0<br>0,8 |

Herstellung:

I Mische die Komponenten von A und erwärme auf 80 °C
II Mische die Komponenten von B bis alle Stoffe gelöst sind (eventuell unter leichtem Erwärmen)
III Gebe II zu I
IV Mische die Komponenten von C und erwärme auf 50 °C
V Rühre IV in I mit hoher Geschwindigkeit bis zur Abkühlung auf 35 °C
VI Gebe D bei 35 °C zu V

Formulierungsbeispiele 3 und 4: Pflanzenschutzformulierungen

| Formulierung Nr. | 3 | 4 |
|---|---|---|
| Inhaltsstoff | Menge des jeweiligen Inhaltsstoffes [Gew.-%] | |
| Atrazin | 43,6 | 43,6 |
| Dispersogen® PSL 100 | - | 1,7 |
| Genapol® LSS | - | 1,6 |
| Dispersogen® LFS | 2,1 | - |
| Propylenglykol | 4,3 | 4,3 |
| Defoamer® SE 57 | 0,6 | 0,6 |
| Kelzan® S (2 Gew.-% in Wasser) | 7,3 | 7,3 |
| Isosorbidcaprylat 1 | 0,3 | 0,2 |

(fortgesetzt)

| Formulierung Nr. | 3 | 4 |
|---|---|---|
| Phenoxyethanol | 1,0 | 1,0 |
| Wasser | ad 100 | ad 100 |

Herstellung:

Der Wirkstoff wird mit den anderen Inhaltsstoffen (außer der Kelzan® S-Lösung) vordispergiert und anschließend einer Feinmahlung unterzogen, bis die mittlere Teilchengröße < 2 Mikrometer beträgt. Anschließend wird die Kelzan® S-Lösung eingerührt.

Formulierungsbeispiel 5: Handgeschirrspülmittel

| Inhaltsstoff | Gew. % |
|---|---|
| Hostapur® SAS 60 | 40,0 |
| (Alkansulfonat, 60 Gew.-% in Wasser) | |
| Hostapur® OS liquid (Sodium C14-16 Alkyl Sulfonate, 40 Gew.-% in Wasser) | 11,0 |
| Genaminox® LA (Dimethyllauraminoxid, 30 Gew.-% in Wasser) | 3,0 |
| Genagen® CAB (Cocoamidopropylbetain, 30 Gew.-% in Wasser) | 3,0 |
| Isosorbidcaprylat 1 | 0,8 |
| Benzyl Alcohol | 0,8 |
| Wasser | ad 100 |

Formulierungsbeispiel 6: Oberflächenreiniger (Allzweckreiniger)

| Inhaltsstoff | Gew. % |
|---|---|
| Hostapur® SAS 60 (Alkansulfonat, 60 Gew.-% in Wasser) | 5,0 |
| Genapol® UD 080 (Undecanol + 8 EO) | 2,0 |
| Genaminox® LA (Dimethyllauraminoxid, 30 Gew.-% in Wasser) | 2,0 |
| Methylisothiazolinone | 0,01 |
| Isosorbidcaprylat 1 | 1,0 |
| Wasser | ad 100 |

Herstellung der Formulierungsbeispiele 5 und 6:

Die Hälfte der Wassermenge wird vorgelegt und die Komponenten werden in der Reihenfolge wie in den bei Formulierungsbeispielen 5 und 6 angegebenen Tabellen aufgeführt eingerührt. Die restliche Menge Wasser wird dann nachgegeben. Es resultieren klare, wässrige Zusammensetzungen.

## Patentansprüche

**1.** Zusammensetzung enthaltend
eine oder mehrere Verbindungen der Formel (I) und eine oder mehrere Verbindungen der Formel (II)

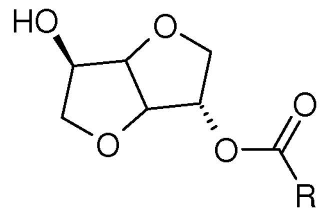

(I)

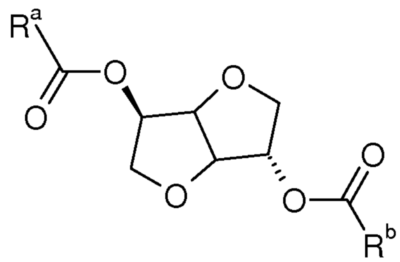

(II)

worin
R, $R^a$ und $R^b$ jeweils unabhängig voneinander lineare oder verzweigte, gesättigte Alkylgruppen mit 5 bis 11, vorzugsweise 7 bis 9 und besonders bevorzugt 7 Kohlenstoffatomen oder lineare oder verzweigte, ein- oder mehrfach ungesättigte Alkenylgruppen mit 5 bis 11, vorzugsweise 7 bis 9 und besonders bevorzugt 7 Kohlenstoffatomen sind,
**dadurch gekennzeichnet, dass** die Gesamtmenge der Verbindungen der Formeln (I) und (II), bezogen auf das Gesamtgewicht der Zusammensetzung, mindestens 60 Gew.-% beträgt,
wobei die Zusammensetzung zusätzlich zu den Verbindungen der Formeln (I) und (II) eine oder mehrere andere Substanzen ausgewählt aus der Gruppe bestehend aus Sorbitol, Sorbitolestern, Sorbitan, Sorbitanestern, Isosorbid und Carbonsäuren enthält
und die OH-Zahl der Mischung aus den Verbindungen der Formeln (I) und (II) und zusätzlich der einen oder den mehreren Verbindungen ausgewählt aus der Gruppe bestehend aus Sorbitol, Sorbitolestern, Sorbitan, Sorbitanestern, Isosorbid und Carbonsäuren kleiner oder gleich 250 ist,
wobei die Zusammensetzung 0,4 bis 0,8 Gewichtsteile an der einen oder den mehreren Verbindungen der Formel (II), bezogen auf 1,0 Gewichtsteile an der einen oder den mehreren Verbindungen der Formel (I), enthält.

2. Zusammensetzung nach Anspruch 1 zur Pilzbekämpfung.

3. Zusammensetzung nach Anspruch 1 oder 2, **dadurch gekennzeichnet, dass** die Reste R, $R^a$ und $R^b$ in den Formeln (I) und (II) jeweils unabhängig voneinander lineare gesättigte Alkylreste mit 7 bis 9 Kohlenstoffatomen sind.

4. Zusammensetzung nach Anspruch 3, **dadurch gekennzeichnet, dass** die Reste R, $R^a$ und $R^b$ in den Formeln (I) und (II) lineare gesättigte Alkylreste mit 7 Kohlenstoffatomen sind.

5. Zusammensetzung nach einem oder mehreren der Ansprüche 1 bis 4, **dadurch gekennzeichnet, dass** sie 0,4 bis 0,8 Gewichtsteile an der einen oder den mehreren Verbindungen der Formel (II), wobei es sich dabei um Isosorbiddicaprylat handelt,
bezogen auf 1,0 Gewichtsteile an der einen oder den mehreren Verbindungen der Formel (I) und vorzugsweise bezogen auf 1,0 Gewichtsteile an Isosorbidmonocaprylat, enthält.

6. Zusammensetzung nach Anspruch 5, **dadurch gekennzeichnet, dass** sie 0,05 bis 0,7, vorzugsweise 0,1 bis 0,6 und besonders bevorzugt 0,2 bis 0,5 Gewichtsteile Isosorbid, bezogen auf 1,0 Gewichtsteile an der einen oder den mehreren Verbindungen der Formel (I) und vorzugsweise bezogen auf 1,0 Gewichtsteile an Isosorbidmonocaprylat, enthält.

7. Zusammensetzung nach einem oder mehreren der Ansprüche 1 bis 4, **dadurch gekennzeichnet, dass** sie 0,001 bis 0,2, vorzugsweise 0,01 bis 0,15 und besonders bevorzugt 0,05 bis 0,13 Gewichtsteile an der einen oder den mehreren Verbindungen der Formel (I), wobei es sich dabei vorzugsweise um Isosorbidmonocaprylat handelt, bezogen auf 1,0 Gewichtsteile an der einen oder den mehreren Verbindungen der Formel (II) und vorzugsweise bezogen auf 1,0 Gewichtsteile an Isosorbiddicaprylat, enthält.

8. Zusammensetzung nach einem oder mehreren der Ansprüche 1 bis 7, **dadurch gekennzeichnet, dass** sie eine oder mehrere andere Substanzen ausgewählt aus der Gruppe bestehend aus Sorbitol, Sorbitolestern, Isosorbid und Carbonsäuren enthält.

9. Zusammensetzung nach einem oder mehreren der Ansprüche 1 bis 8, **dadurch gekennzeichnet, dass** die OH-Zahl der Mischung aus den Verbindungen der Formeln (I) und (II) und zusätzlich der einen oder den mehreren Verbindungen ausgewählt aus der Gruppe bestehend aus Sorbitol, Sorbitolestern, Sorbitan, Sorbitanestern, Isosorbid und Carbonsäuren kleiner oder gleich 230, bevorzugt kleiner oder gleich 210 und insbesondere bevorzugt kleiner oder gleich 100 ist.

10. Zusammensetzung nach einem oder mehreren der Ansprüche 1 bis 9, **dadurch gekennzeichnet, dass** sie jeweils eine oder mehrere Verbindungen der Formeln (I) und (II) und zusätzlich einen oder mehrere Sorbitanester aus Sorbitan und Carbonsäuren $R^cCOOH$, vorzugsweise ausgewählt aus Sorbitanestern aus 1,4- und/oder 1,5-Sorbitan und Carbonsäuren $R^cCOOH$, wobei $R^c$ eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, enthält, und das Gewichtsverhältnis der Verbindungen der Formeln (I) und (II) gemeinsam zu dem einen oder den mehreren soeben genannten Sorbitanestern von 70 : 30 bis 100 : 0, vorzugsweise von 80 : 20 bis 100 : 0, besonders bevorzugt von 90 : 10 bis 100 : 0 und insbesondere bevorzugt von 95 : 5 bis 100 : 0 ist.

11. Zusammensetzung nach Anspruch 10, **dadurch gekennzeichnet, dass** der eine oder die mehreren Sorbitanester aus Sorbitan und Carbonsäuren $R^cCOOH$ ausgewählt sind aus Sorbitanestern aus Sorbitan und Caprylsäure, vorzugsweise ausgewählt sind aus Sorbitanestern aus 1,4- und/oder 1,5- Sorbitan und Caprylsäure und besonders bevorzugt ausgewählt sind aus der Gruppe bestehend aus Sorbitanmonocaprylat und Sorbitandicaprylat.

12. Verwendung einer Zusammensetzung nach einem oder mehreren der Ansprüche 1 bis 11 zur Herstellung einer kosmetischen, dermatologischen oder pharmazeutischen Zusammensetzung, einer Pflanzenschutzformulierung, eines Wasch- oder Reinigungsmittels oder eines Farb- oder Anstrichmittels.

13. Verwendung einer Zusammensetzung nach einem oder mehreren der Ansprüche 1 bis 11 als antimikrobieller Wirkstoff gegen Pilze.

14. Verwendung einer Zusammensetzung nach einem oder mehreren der Ansprüche 1 bis 11 als antimikrobieller Wirkstoff gegen Hefen.

## Claims

1. A composition comprising
one or more compounds of the formula (I) and one or more compounds of the formula (II)

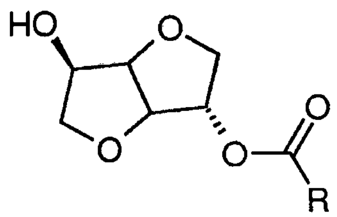

(I)

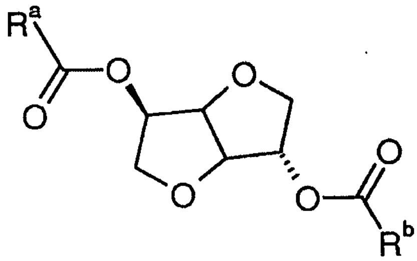

(II)

in which
R, $R^a$ and $R^b$ are each independently of one another straight-chain or branched saturated alkyl groups having 5 to 11, preferably 7 to 9 and particularly preferably 7 carbon atoms or straight-chain or branched mono- or polyunsaturated alkenyl groups having 5 to 11, preferably 7 to 9 and particularly preferably 7 carbon atoms,
wherein the total amount of the compounds of the formulae (I) and (II), based on the total weight of the composition, is at least 60% by weight, wherein the composition, in addition to the compounds of the formulae (I) and (II), comprises one or more other substances selected from the group consisting of sorbitol, sorbitol esters, sorbitan, sorbitan esters, isosorbide and carboxylic acids,
and the hydroxyl value of the mixture of the compounds of the formulae (I) and (II) and additionally the one or more compounds selected from the group consisting of sorbitol, sorbitol esters, sorbitan, sorbitan esters, isosorbide and carboxylic acids is smaller than or equal to 250,
wherein the composition comprises from 0.4 to 0.8 part by weight of the one or more compounds of the formula (II), based on 1.0 part by weight of the one or more compounds of the formula (I).

2. The composition as claimed in claim 1, for controlling fungi.

3. The composition as claimed in claim 1 or 2, wherein the radicals R, $R^a$ and $R^b$ in the formulae (I) and (II) are each independently of one another straight-chain saturated alkyl radicals having 7 to 9 carbon atoms.

4. The composition as claimed in claim 3, wherein the radicals R, $R^a$ and $R^b$ in the formulae (I) and (II) are straight-chain saturated alkyl radicals having 7 carbon atoms.

5. The composition as claimed in one or more of claims 1 to 4, comprising from 0.4 to 0.8 part by weight of the one or more compounds of the formula (II), which is isosorbide dicaprylate,
based on 1.0 part by weight of the one or more compounds of the formula (I) and preferably based on 1.0 part by weight of isosorbide monocaprylate.

6. The composition as claimed in claim 5, comprising from 0.05 to 0.7, preferably from 0.1 to 0.6 and particularly preferably from 0.2 to 0.5 part by weight of isosorbide, based on 1.0 part by weight of the one or more compounds of the formula (I) and preferably based on 1.0 part by weight of isosorbide monocaprylate.

7. The composition as claimed in one or more of claims 1 to 4, comprising from 0.001 to 0.2, preferably from 0.01 to 0.15 and particularly preferably from 0.05 to 0.13 part by weight of the one or more compounds of the formula (I), which is preferably isosorbide monocaprylate, based on 1.0 part by weight of the one or more compounds of the formula (II) and preferably based on 1.0 part by weight of isosorbide dicaprylate.

8. The composition as claimed in one or more of claims 1 to 7, comprising, one or more other substances selected from the group consisting of sorbitol, sorbitol esters, isosorbide and carboxylic acids.

9. The composition as claimed in one or more of claims 1 to 8, wherein the hydroxyl value of the mixture of the compounds of the formulae (I) and (II) and additionally the one or more compounds selected from the group consisting of sorbitol, sorbitol esters, sorbitan, sorbitan esters, isosorbide and carboxylic acids is smaller than or equal to 230, preferably smaller than or equal to 210 and especially preferably smaller than or equal to 100.

10. The composition as claimed in one or more of claims 1 to 9, comprising in each case one or more compounds of the formula (I) and (II) and additionally one or more sorbitan esters of sorbitan and carboxylic acids $R^cCOOH$, preferably selected from sorbitan esters from 1,4- and/or 1,5-sorbitan and carboxylic acids $R^cCOOH$ where $R^c$ is a straight-chain or branched saturated alkyl group having 5 to 11, preferably 7 to 9 and particularly preferably 7 carbon atoms or a straight-chain or branched mono- or polyunsaturated alkenyl group having 5 to 11, preferably 7 to 9 and particularly preferably 7 carbon atoms, and where the weight ratio of the compounds of the formulae (I) and (II) together to the one or more sorbitan esters just mentioned is from 70 : 30 to 100 : 0, preferably from 80 : 20 to 100 : 0, particularly preferably from 90 : 10 to 100 : 0 and especially preferably from 95 : 5 to 100 : 0.

11. The composition as claimed in claim 10, wherein the one or more sorbitan esters of sorbitan and carboxylic acids $R^cCOOH$ are selected from sorbitan esters of sorbitan and caprylic acid, preferably selected from sorbitan esters of 1,4- and/or 1,5-sorbitan and caprylic acid and particularly preferably selected from the group consisting of sorbitan monocaprylate and sorbitan dicaprylate.

**12.** The use of a composition as claimed in one or more of claims 1 to 11 for producing a cosmetic, dermatological or pharmaceutical composition, a crop protection formulation, a washing or cleaning composition or a paint or coating.

**13.** The use of a composition as claimed in one or more of claims 1 to 11 as an antimicrobially active agent against fungi.

**14.** The use of a composition as claimed in one or more of claims 1 to 11 as an antimicrobially active agent against yeasts.

**Revendications**

**1.** Composition contenant
un ou plusieurs composés de formule (I) et un ou plusieurs composés de formule (II)

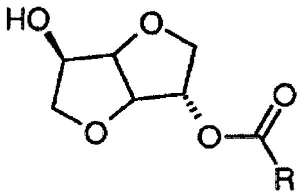

(I)

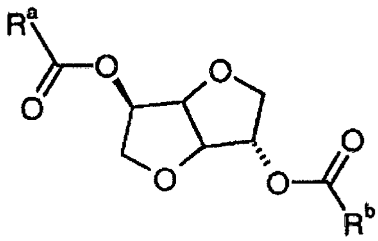

(II)

formules dans lesquelles
R, $R^a$ et $R^b$ représentent chacun indépendamment des groupes alkyle linéaires ou ramifiés, saturés, ayant de 5 à 11, de préférence de 7 à 9 et de façon particulièrement préférée 7 atomes de carbone, ou des groupes alcényle linéaires ou ramifiés, une ou plusieurs fois insaturés, ayant de 5 à 11, de préférence de 7 à 9 et de façon particulièrement préférée 7 atomes de carbone,
**caractérisée en ce que** la quantité totale des composés de formules (I) et (II), par rapport au poids total de la composition, est d'au moins 60 % en poids,
la composition contenant, en plus des composés de formule (I) et (II), une ou plusieurs autres substances choisies dans le groupe constitué par le sorbitol, des esters de sorbitol, le sorbitane, des esters de sorbitane, l'isosorbide et des acides carboxyliques,
et l'indice de groupes OH du mélange des composés de formules (I) et (II) et additionnellement desdits un ou desdits plusieurs composés choisis dans le groupe constitué par le sorbitol, des esters de sorbitol, le sorbitane, des esters de sorbitane, l'isosorbide et des acides carboxyliques, étant inférieur ou égal à 250,
la composition contenant 0,4 à 0,8 partie en poids dudit un ou desdits plusieurs composés de formule (II), par rapport à 1,0 partie en poids dudit un ou desdits plusieurs composés de formule (I).

**2.** Composition selon la revendication 1, destinée à la lutte contre des champignons.

**3.** Composition selon la revendication 1 ou 2, **caractérisée en ce que** les radicaux R, $R^a$ et $R^b$ dans les formules (I) et (II) représentent chacun indépendamment des radicaux alkyle linéaires saturés ayant de 7 à 9 atomes de carbone.

**4.** Composition selon la revendication 3, **caractérisée en ce que** les radicaux R, $R^a$ et $R^b$ dans les formules (I) et (II) sont des radicaux alkyle linéares saturés ayant 7 atomes de carbone.

**5.** Composition selon une ou plusieurs des revendications 1 à 4, **caractérisée en ce qu'**elle contient 0,4 à 0,8 partie en poids dudit un ou desdits plusieurs composés de formule (II), pour ce qui est desquels il s'agit de dicaprylate d'isosorbide,
par rapport à 1,0 partie en poids dudit un ou desdits plusieurs composés de formule (I) et de préférence par rapport

à 1,0 partie en poids de monocaprylate d'isosorbide.

6. Composition selon la revendication 5, **caractérisée en ce qu'**elle contient 0,05 à 0,7, de préférence 0,1 à 0,6 et de façon particulièrement préférée 0,2 à 0,5 partie en poids d'isosorbide, par rapport à 1,0 partie en poids dudit un ou desdits plusieurs composés de formule (I) et de préférence par rapport à 1,0 partie en poids de monocaprylate d'isosorbide.

7. Composition selon une ou plusieurs des revendications 1 à 4, **caractérisée en ce qu'**elle contient 0,001 à 0,2, de préférence 0,01 à 0,15 et de façon particulièrement préférée 0,05 à 0,13 partie en poids dudit un ou desdits plusieurs composés de formule (I), pour ce qui est desquels il s'agit de préférence de monocaprylate d'isosorbide, par rapport à 1,0 partie en poids dudit un ou desdits plusieurs composés de formule (II) et de préférence par rapport à 1,0 partie en poids de dicaprylate d'isosorbide.

8. Composition selon une ou plusieurs des revendications 1 à 7, **caractérisée en ce qu'**elle contient une ou plusieurs autres substances choisies dans le groupe constitué par le sorbitol, des esters de sorbitol, l'isosorbide et des acides carboxyliques,

9. Composition selon une ou plusieurs des revendications 1 à 8, **caractérisée en ce que** l'indice de groupes OH du mélange des composés de formules (I) et (II) et additionnellement dudit un ou desdits plusieurs composés choisis dans le groupe constitué par le sorbitol, des esters de sorbitol, le sorbitane, des esters de sorbitane, l'isosorbide et des acides carboxyliques, est inférieur ou égal à 230, de préférence inférieur ou égal à 210 et de façon particulièrement préférée inférieur ou égal à 100.

10. Composition selon une ou plusieurs des revendications 1 à 9, **caractérisée en ce qu'**elle contient un ou plusieurs de chacun des composés de formules (I) et (II) et en plus un ou plusieurs esters de sorbitane à base de sorbitane et d'acides carboxyliques $R^cCOOH$, de préférence choisis parmi des esters de sorbitane à base de 1,4- et/ou 1,5-sorbitane et d'acides carboxyliques $R^cCOOH$, $R^c$ étant un groupe alkyle linéaire ou ramifié, saturé, ayant de 5 à 11, de préférence de 7 à 9 et de façon particulièrement préférée 7 atomes de carbone, ou un groupe alcényle linéaire ou ramifié, une ou plusieurs fois insaturé, ayant de 5 à 11, de préférence de 7 à 9 et de façon particulièrement préférée 7 atomes de carbone, et le rapport pondéral des composés de formules (I) et (II) conjointement avec ledit un ou lesdits plusieurs esters de sorbitane qui viennent d'être nommés vaut de 70:30 à 100:0, de préférence de 80:20 à 100:0, de façon particulièrement préférée de 90:10 à 100:0 et de façon plus particulièrement préférée de 95:5 à 100:0.

11. Composition selon la revendication 10, **caractérisée en ce que** ledit un ou lesdits plusieurs esters de sorbitane à base de sorbitane et d'acides carboxyliques $R^cCOOH$ sont choisis parmi les esters de sorbitane à base de sorbitane et d'acide caprylique, de préférence sont choisis parmi les esters de sorbitane à base de 1,4- et/ou 1,5-sorbitane et d'acide caprylique et de façon particulièrement préférée sont choisis dans le groupe constitué par le monocaprylate de sorbitane et le dicaprylate de sorbitane.

12. Utilisation d'une composition selon une ou plusieurs des revendications 1 à 11, pour la production d'un composition cosmétique, dermatologique ou pharmaceutique, d'une composition phytosanitaire, d'un produit de lavage ou de nettoyage ou d'un produit colorant ou enduit.

13. Utilisation d'une composition selon une ou plusieurs des revendications 1 à 11, en tant que substance active antimicrobienne contre des champignons.

14. Utilisation d'une composition selon une ou plusieurs des revendications 1 à 11, en tant que substance active antimicrobienne contre des levures.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010108738 A2
- DE 102009022444
- DE 102009022445
- JP 8187070 A
- JP 8173787 A